# EUROPEAN PATENT SPECIFICATION

(11) **EP 4 006 549 B1**
(45) Date of publication and mention of the grant of the patent: **02.04.2025**
(21) Application number: 20846114.5
(22) Date of filing: 30.07.2020
(51) Int. Cl.: G01N 33/543, G01N 35/02, G01N 21/25, G01N 35/04, G01N 35/10, G01N 35/00

(54) **DEVICE FOR SIMULTANEOUS ANALYSIS OF MULTIPLE BIOMARKERS AND METHOD FOR SIMULTANEOUS ANALYSIS OF MULTIPLE BIOMARKERS**
VORRICHTUNG ZUR GLEICHZEITIGEN ANALYSE MEHRERER BIOMARKER UND VERFAHREN ZUR GLEICHZEITIGEN ANALYSE MEHRERER BIOMARKER
DISPOSITIF D'ANALYSE SIMULTANÉE DE MULTIPLES BIOMARQUEURS ET PROCÉDÉ D'ANALYSE SIMULTANÉE DE MULTIPLES BIOMARQUEURS

(30) Priority: 30.07.2019 KR 20190092556; 20.11.2019 KR 20190149799
(43) Date of publication of application: 01.06.2022
(73) Proprietor: PCL, Inc., Seoul 08510 (KR)
(72) Inventor: KIM, So Youn, Seoul 06600 (KR)
(74) Representative: Studio Torta S.p.A.
(86) International application number: PCT/KR2020/010077
(87) International publication number: WO 2021/020910

(56) References cited:
- KR-A- 20130 060 560
- KR-A- 20130 119 006
- KR-A- 20160 041 750
- KR-A- 20170 050 399
- US-A1- 2002 112 541
- US-A1- 2011 312 105
- US-A1- 2018 306 829
- US-A1- 2019 168 220

## Description

### CROSS-REFERENCE TO RELATED PATENT APPLICATION

This application claims the benefit of Korean Patent Application No. 10-2019-0092556, filed on July 30, 2019, No. 10-2019-0149799. Filed on November 20, 2019 and No. 10-2020-0095516, filed on July 30, 2020, in the Korean Intellectual Property Office, the disclosure of which is incorporated herein in its entirety by reference.

### BACKGROUND OF THE INVENTION

### 1. Field of the Invention

The present disclosure relates to a multiple biomarker simultaneous analysis apparatus and method.

### 2. Description of the Related Art

Viruses are pathogens smaller than 1 µm in size and cannot be observed even with an optical microscope, and an electron microscope is required to see viruses with the naked eye. In addition, a drug control method that directly acts on viruses has not yet been developed. In the past, electron microscopy and serological methods were mainly used for virus diagnosis. In a method using an electron microscope, the presence of a virus can be confirmed, but it is impossible to diagnose a species based on its morphological characteristics. Among serological methods, an enzyme-linked immunosorbent assay has about 1000 times lower detection sensitivity than the most commonly used diagnostic method or polymerase chain reaction (PCR) diagnostic method, and diagnosis often fails due to an unexpected non-specific reaction between an antibody and a test sample.

For this reason, precise and rapid diagnosis is a prerequisite for the management of viral diseases. Meanwhile, in a reverse transcription-polymerase chain reaction (RT-PCR) method, which is commonly used for precise diagnosis in plant viruses, the primer used determines the specificity and precision of diagnosis. In the case of commonly used primers, these primers are designed for the purpose
of virus gene cloning and are not species-specific in many cases. Because there are cases where non-specific products are produced by plants or other viruses, they are often unsuitable for use in diagnosis. In addition, an RT-PCR method is performed using various types of primers so as to diagnose several types of viruses from the same sample such that a large amount of diagnostic cost and labor is required.

Documents US2011312105, US2018306829 and US2002112541 disclose multiple biomarker simultaneous analysis apparatuses of the known type.

Meanwhile, because, in a diagnostic device according to the related art, a reaction is performed by adding a reaction reagent, a detection reagent, *etc.* to a sample, errors occur at a relatively high frequency in the detection result due to each of the added reagents. A process of performing a plurality of repeated experiments was required for accurate diagnosis. If it is possible to prevent the occurrence of errors due to the added reagent, the cost and time required for diagnosis may be reduced.

### SUMMARY OF THE INVENTION

The present disclosure provides an analysis apparatus capable of detecting multiple biomarkers only by analyzing a single tip as binding materials capable of being specifically bound to different detection target materials are formed in a tip.

The present disclosure also provides an analysis apparatus in which, as binding materials capable of being specifically bound to a detection target are formed in a tip and an analysis process is performed while the tip is moved, the occurrence of analysis errors due to components other than a sample may be significantly reduced.

The present disclosure also provides an analysis apparatus in which an optical signal generated outside a cartridge is detected, compared to an analysis apparatus according to the related art that detects an optical signal generated within a well of the cartridge, so that the occurrence of interference of the optical signal due to external factors such as contamination and residues may be significantly reduced.

The present disclosure also provides an analysis apparatus in which the inconvenience of using different tips to detect different detection target materials and a plurality of analysis processes are required, may be eliminated.

The present disclosure also provides a method, whereby simultaneous analysis of multiple biomarkers may be performed using the analysis apparatus.

According to appended claim 1 there is provided a cartridge 100 comprising a plurality of wells and a tip 110, and wherein the plurality of wells comprise a sample well 101 configured to accommodate a sample S containing a detection target material T, and a reaction well 105 configured to accommodate a first binding material 105a, which is specifically configured to be bound to the detection target material T and to which a marker R is coupled, and the tip 110 is configured to be inserted into the at least one plurality of wells and a second binding material 111 that is specifically configured to be bound to the detection target material T is coupled to one end of the tip 110; a cartridge mounting portion 210 in which the cartridge 100 is installed; a tip coupling portion 220 coupled to the tip 110 and movable on a predetermined movement trajectory while being coupled to the tip 110; an optical unit 230 radiating light toward the tip 110 and receiving light generated from the tip 110 according to radiation of light; and a processing unit 240 determining whether the detection target material T is present in the sample S according to a predetermined method using the light received by the optical unit 230.

The multiple biomarker simultaneous analysis apparatus further includes: a cartridge mounting portion moving portion which is coupled to the cartridge mounting portion 210 and configured to move the cartridge mounting portion 210 so that the at least one well of the cartridge 100 and the tip coupling portion 220 are aligned with each other; and a tip coupling portion moving portion 260 which is coupled to the tip coupling portion 220 and configured to move the tip coupling portion 220 on the predetermined movement trajectory.

The multiple biomarker simultaneous analysis apparatus further includes: a tip coupling portion mounting base 270 in which the tip coupling portion 220 is installed; and a mounting base support portion 280 which is installed on a moving rail 281 having one end extending in a vertical direction and coupled to an outside of the tip coupling portion mounting base 270, wherein the tip coupling portion moving portion 260 includes: a first tip coupling portion moving portion 261, which is installed on the tip coupling portion mounting base 270 and is configured to provide a driving force so that the tip coupling portion mounting base 270 rotates between a position where the tip coupling portion 220 installed on the tip coupling portion mounting base 270 is aligned in a line with the at least one well of the cartridge 100 in the vertical direction and a position where the tip coupling portion 220 is aligned in a line with an optical path on which light is radiated by the optical unit 230; and a second tip coupling portion moving portion 262, which is installed on the mounting base support portion 280 and is configured to provide a driving force so that the tip coupling portion mounting base 270 vibrates in the vertical direction with a predetermined amplitude, wherein the position where the tip coupling portion installed on the tip coupling portion mounting base is aligned in a line with the plurality of wells is a position perpendicular to ground, and the position where the tip coupling portion is aligned in a line with an optical path is a position parallel to the ground.

The cartridge mounting portion moving portion is configured to move the cartridge mounting portion 210 in a direction perpendicular to the vertical direction.

The cartridge 100 may further include: a tip well 102 into which the tip 110 is inserted; and at least one absorption pad well 104 in which an absorption pad 104a is installed; at least one washing well 106, 107, 108, and 109 in which a washing solution is accommodated; and a punching tip well 103 into which a punching tip 120 for punching sealing portions 104b, 105b, 106b, 107b, 108b, and 109b of the absorption pad well 104, the reaction well 105, and the at least one washing well 106, 107, 108, and 109.

The tip coupling portion 220 may be selectively coupled to the tip 110 or the punching tip 120.

At least one binding material that is specifically bound to different detection target materials from the detection target material T may be further coupled to a lower surface of the tip 110 to which the second binding material 111 is coupled.

The multiple biomarker simultaneous analysis apparatus may further include an intermediate portion 231, which is installed between the optical unit 230 and the tip coupling portion mounting base 270 and has at least one through hole 231a formed on an optical path on which light is radiated by the optical unit 230.

The processing unit may determine whether a plurality of detection target materials T are present in the sample S using a number of combinations of intensities of light received by the optical unit 230 for different detection target materials.

The processing unit 240 may determine whether the plurality of detection target materials T are present in the sample S using positions of binding materials coupled to the lower surface of the tip 110 and the number of combinations of intensities of light received at the positions.

The processing unit 240 may determine amounts of the plurality of detection target materials T contained in the sample S using positions of binding materials coupled to the lower surface of the tip 110 and the number of combinations of intensities of light received at the positions.

The cartridge mounting portion 210 includes a plurality of seating portions 211 in which the cartridge 100 is installed, the plurality of seating portions 211 being arranged in parallel to each other, and the tip coupling portion 220 may be formed in a number matching a number of the seating portions 211 one-to-one.

According to appended claim 9, there is provided a multiple biomarker simultaneous analysis method using the multiple biomarker simultaneous analysis apparatus of claim 2, the multiple biomarker simultaneous analysis method including: (a) injecting the sample S into the sample well 101 of the cartridge 100; (b) aligning the tip well 102 of the cartridge 100 and the tip coupling portion 220 in a line in a vertical direction; (c) coupling the tip coupling portion 220 to the tip 110; (d) aligning the sample well 101 of the cartridge 100 and the tip coupling portion 220 in a line in the vertical direction and injecting the tip 110 into the sample S accommodated in the sample well 101; (e) aligning the reaction well 105 of the cartridge 100 and the tip coupling portion 220 in a line in the vertical direction and injecting the tip 110 into a reaction sample 105c accommodated in the reaction well 105; (f) aligning one of the at least one washing well 106, 107, 108, and 109 of the cartridge 100 and the tip coupling portion 220 in a line in the vertical direction and injecting the tip 110 into a washing solution accommodated in the one washing well; (g) aligning the moisture absorption pad well 104 of the cartridge 100 and the tip coupling portion 220 in a line in the vertical direction and injecting the tip 110 into the moisture absorption pad 104a provided in the moisture absorption pad well 104; (h) rotating the tip coupling portion mounting base 270 so that the optical path on which light is radiated by the optical unit 230 and the tip 110 are aligned in a line; (i) radiating light toward the tip 110 using the optical unit 230 and receiving the light generated from the tip 110 by the optical unit 230; and (j) determining whether the detection target material T is present in the sample S using intensity of light received by the optical unit 230, wherein the determining is performed by using the processing unit 240.

The multiple biomarker simultaneous analysis method may further include, after (a) and before (b), (a1) aligning the punching tip well 103 of the cartridge 100 and the tip coupling portion 220 in a line in the vertical direction; (a2) coupling the tip coupling portion 220 to the punching tip 120; and (a3) aligning each of the moisture absorption pad well 104, the reaction well 105, and the at least one washing well 106, 107, 108, and 109 of the cartridge 100 with the tip coupling portion 220 in a line in the vertical direction and punching the sealing portion 104b of the moisture absorption pad well 104, the sealing portion 105b of the reaction well 105, and the sealing portions 106b, 107b, 108b, and 109b of the at least one washing well 106, 107, 108, and 109.

Any one or more of (e), (f), and (g) may further include vibrating the tip coupling portion 220 in the vertical direction with a predetermined amplitude while being injected into a well of the cartridge 100.

### BRIEF DESCRIPTION OF THE DRAWINGS

The above and other features and advantages of the present invention will become more apparent by describing in detail exemplary embodiments thereof with reference to the attached drawings in which:
FIGS. 1 and 2 are views for describing a cartridge mounted on a multiple biomarker simultaneous analysis apparatus according to an embodiment of the present disclosure;
FIGS. 3 and 4 are perspective views illustrating a multiple biomarker simultaneous analysis apparatus according to an embodiment of the present disclosure;
FIG. 5 is a view for describing a cartridge mounting portion of the multiple biomarker simultaneous analysis apparatus of FIG. 3;
FIG. 6 is a view for describing a tip coupling portion of the multiple biomarker simultaneous analysis apparatus of FIG. 3, and a tip coupling portion mounting base and a mounting base support in which the tip binding portion is installed;
FIG. 7 is a view for describing the operation relationship between the cartridge mounting portion, the tip coupling portion, the tip coupling portion mounting base, and the mounting base support portion;
FIG. 8 is a view for schematically describing a process of analyzing a sample injected into the cartridge using a multiple biomarker simultaneous analysis apparatus according to an embodiment of the present disclosure;
FIGS. 9 through 21 are views for describing a process of analyzing a sample injected into a cartridge using a multiple biomarker simultaneous analysis apparatus according to an embodiment of the present disclosure;

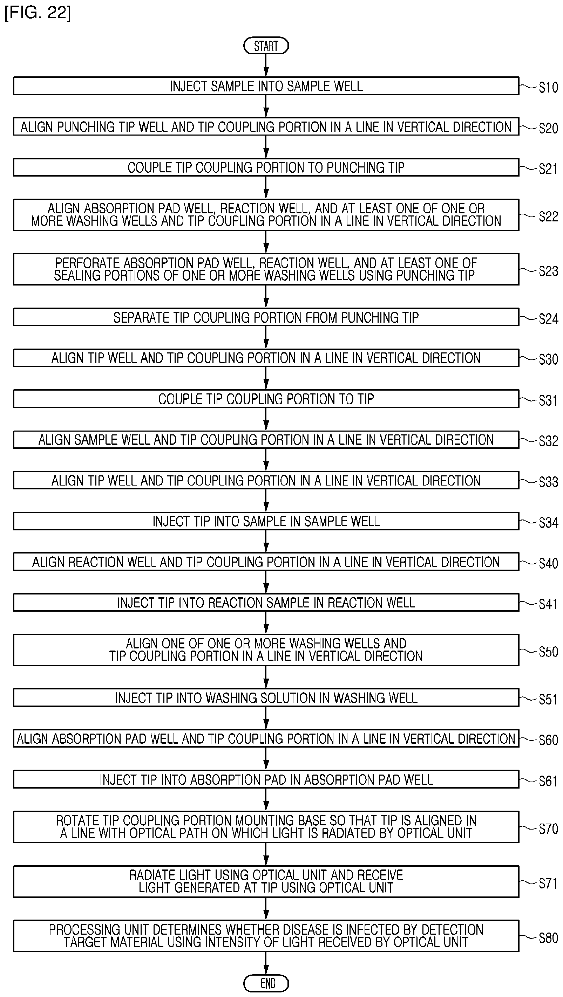

FIG. 22 is a flowchart illustrating a multiple biomarker simultaneous analysis method according to an embodiment of the present disclosure; and
FIGS. 23 through 25 are views for describing the determination of the presence or absence of a biomarker in a sample using light received from an optical unit after radiating light toward a tip in a multiple biomarker simultaneous analysis apparatus according to an embodiment of the present disclosure.

### DETAILED DESCRIPTION OF THE INVENTION

Hereinafter, a multiple biomarker simultaneous analysis apparatus 200 according to an embodiment of the present disclosure will be described in more detail with reference to the accompanying drawings.

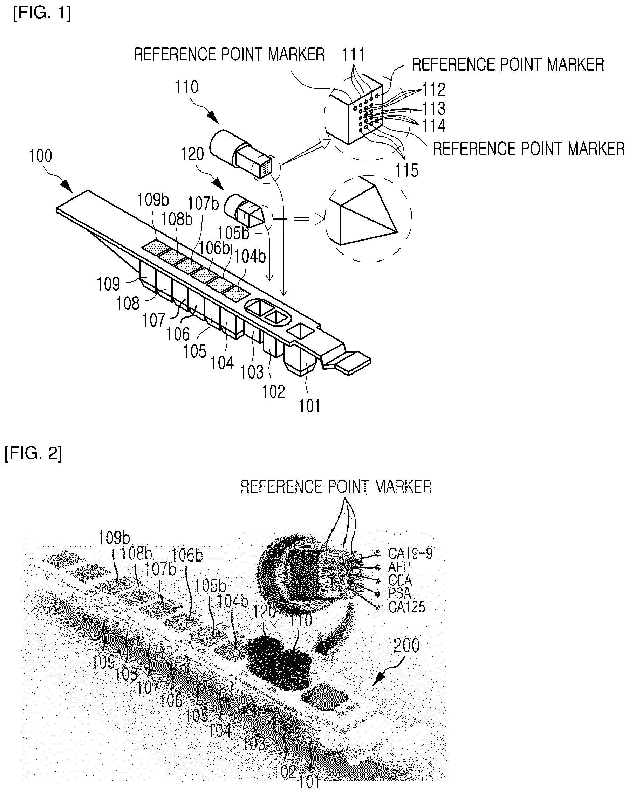

First, a cartridge 100, which is mounted on the multiple biomarker simultaneous analysis apparatus 200 and analyzed by the multiple biomarker simultaneous analysis apparatus 200, will be described in detail with reference to FIGS. 1 and 2.

FIG. 1 is a view for describing a cartridge 100 that may be mounted on a multiple biomarker simultaneous analysis apparatus 200 according to an embodiment of the present disclosure.

Referring to FIG. 1, the cartridge 100 according to an embodiment of the present disclosure may include a sample well 101, a tip well 102, a punching tip well 103, a moisture absorption pad well 104, a reaction well 105, a first washing well 106, a second washing well 107, a third washing well 108, and a fourth washing well 109.

A sample S of an analyte is injected into the sample well 101, and the sample S may be a blood sample of the analyte, but the sample S may be extracted from a person to be tested, such as sweat, saliva, runny nose, sputum, urine, feces, carriers, *etc.* As long as the sample S may contain a detection target material T capable of determining the presence of the detection target material T in the sample S, embodiments of the present disclosure are not limited thereto.

A tip 110 is inserted into the tip well 102.

The front end of the tip 110 includes a surface on which binding materials specifically bound to the detection target material T are sol-gel-spotted. Specific details on the sol-gel spotting of the binding materials contain the contents of the present applicant's prior patent applications KR 10-2011-0039535 (April 27, 2011), PCT-KR2011-003105 (April 27, 2011), KR 10-2006-0008926 (January 27, 2006), PCT-KR2007-000390 (January 23, 2007), KR 10-2013-0047564 (April 29, 2013), PCT-KR2013-003657 (April 26, 2013), and KR 10-2018-0125995 (October 22, 2018).

The tip 110 is detachably inserted into the tip well 102, and a component capable of being bound to a detection target material (biomarker) included in the target sample S by an immunological or non-immunological method is coupled to the lower surface of the tip 110 using a sol-gel spot. At this time, the binding materials coupled to the lower surface of the tip 110 may be an antigen, an antibody, or an aptamer, but as long as the binding materials are materials capable of being specifically bound to the detection target material, embodiments of the present disclosure are not specifically limited thereto.

The binding materials coupled to the lower surface of the tip 110 are, for example, Anti-HIV 1/2 detecting antigen, Anti-HCV detecting antigen, HBsAg detecting antibody, Influenza A Antibody, Influenza B Antibody, RSV Antibody, SARS-CoV -2 Antibody, SARS-CoV-2 Antigen, CA19-9 antibody, AFP antibody, CEA antibody, PSA antibody, CA 125 antibody, CA 15-3 antibody, CA 242 antibody, CYFRA21-1 antibody, SCCA antibody, NSE antibody, HE4 antibody, Canine-based CDV, CPV, CPIV, CAV-2 and CCoV, Feline-based FCV, FPV, FVR (FHV), FIP (FCoV) and Chl. Felis, *etc.,* but embodiments of the present disclosure are not limited thereto.

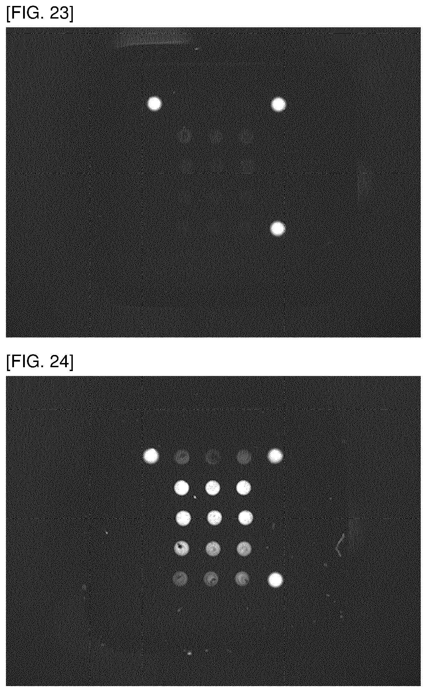

FIG. 1 shows a state in which a second binding material 111 that is a CA19-9 antibody that is specifically bound to CA19-9, a third binding material 112 that is an AFP antibody that is specifically bound to AFP, a fourth binding material 113 that is a CEA antibody that is specifically bound to CEA, a fifth binding material 114 that is a PSA antibody that is specifically bound to PSA and a sixth binding material 115 that is a CA125 antibody that is specifically bound to CA125 are coupled to the lower surface of one tip 110 using the sol-gel-spot (an arrangement of 5 × 3 is shown). In addition, reference point markers may be formed on the lower surface of the tip 110. As shown in FIGS. 1 and 23, the reference point markers may be formed outside the arrangement of the second to sixth bonding materials, and when light is radiated by an optical unit 230, an optical signal of a predetermined intensity may be generated. The intensity of the optical signal generated at a position where the reference point markers are formed is set as a reference light intensity, and is compared with the intensity of an optical signal generated at positions where other bonding materials are formed later, so that analysis may be performed at a position where the corresponding binding materials are formed.

A single number or a plurality of bonding materials may be coupled to the lower surface of the tip 110. Also, a single type of bonding material or a plurality of types of bonding materials may be coupled to the lower surface of the tip 110. Specifically, the bonding materials coupled to the lower surface of the tip 110 are arranged in one column or one row for each type of the bonding materials, and the arrangement of all bonding materials is 2 × 2, 3 × 3, 4 × 4, 5 × 5, *etc.* However, embodiments of the present disclosure are not limited thereto. That is, as long as all binding materials are coupled to the lower surface of the tip 110 and may be bound to the detection target material included in the sample S through supporting of the sample S, the shape of the arrangement is not limited. In another embodiment of the present disclosure, in order to minimize the error of an analysis method through a triplet test, one binding material may be coupled to the lower surface of the tip 110 in an arrangement of 3 rows per one binding material.

In addition, in another embodiment of the present disclosure, the binding materials coupled to the lower surface of the tip 110 may include two or more selected from the group consisting of Influenza A Antibody, Influenza B Antibody, RSV Antibody, and SARS-CoV2 Antibody.

In addition, in another embodiment of the present disclosure, the binding materials coupled to the lower surface of the tip 110 may include two or more selected from the group consisting of CA19-9 antibody, AFP antibody, CEA antibody, PSA antibody, and CA 125 antibody (see FIG. 1).

In addition, in another embodiment of the present disclosure, the binding materials coupled to the lower surface of the tip 110 may include two or more selected from the group consisting of CA 15-3 antibody, CA 242 antibody, CYFRA21-1 antibody, SCCA antibody, NSE antibody, and HE4 antibody.

The detection target material (biomarker) that is specifically bound to the binding material coupled to the lower surface of the tip 110 may be, for example, a nucleic acid, a peptide, a protein, a small molecule material, a virus, an influenza virus, or a cell. However, embodiments of the present disclosure are not limited thereto.

At the rear end of the tip 110, a tip coupling hole 110a for coupling with a tip coupling portion 220 to be described later may be formed at the rear end of the tip 110, and the tip coupling portion 220 is inserted into the tip coupling hole 110a, so that coupling between the tip coupling portion 220 and the tip 110 may be made.

A punching tip 120 is inserted into a punching tip well 103.

The punching tip 120 is detachably inserted into the punching tip well 102, and the lower surface of the punching tip 120 is provided with a sharp tip so that the punching tip 120 is configured to punch (perforate) at least one sealing portion among a sealing portion 104b that covers the upper portion of the moisture absorption pad well 104, a sealing portion 105b that covers the upper portion of the reaction well 105, a sealing portion 106b that covers the upper portion of the first washing well 106, a sealing portion 107b that covers the upper portion of the second washing well 107, a sealing portion 108b that covers the upper portion of the third washing well 108, and a sealing portion 109b that covers the upper portion of the fourth washing well 109.

Before being mounted on the multiple biomarker simultaneous analysis apparatus 200 and analysis is performed, the sample well 101, the other wells 104 through 109 except for the sample well 101, the tip well 102, and the punching tip well 103 are sealed by the sealing portions 104b through 109b so as to prevent deterioration or deterioration of performance through reaction with external moisture and air, and analysis of the sample S may be performed by punching the sealing portion using the punching tip 120.

A punching tip coupling hole 120a for coupling with a tip coupling portion 220 is formed at the rear end of the punching tip 120, and the tip coupling portion 220 is inserted into the punching tip coupling hole 120a so that coupling between the tip coupling portion 220 and the punching tip 120 may be made.

The moisture absorption pad well 104 is provided with a moisture absorption pad 104a. A sponge may be applied as the moisture absorption pad 104a. However, if the moisture absorption pad 104a is made of a material capable of absorbing moisture, the material of the moisture absorption pad 104a is not particularly limited thereto.

The moisture absorption pad well 104 is a portion into which the tip 110 is inserted after supporting the sample S, supporting a reaction sample 105c and supporting a washing solution of the tip 110, and as the tip 110 is inserted into the moisture absorption pad well 104, moisture or other unreacted biological material remaining on the lower surface of the tip 110 is removed. Thus, when light is radiated by the optical unit 230, clearer light receiving sensitivity may be achieved.

The reaction sample 105c is stored in the reaction well 105. The reaction sample 105c includes a first binding material 105a that is specifically bound to the detection target material T, like the binding materials coupled to the lower surface of the tip 110. A marker R is bound to the first binding material 105a, and the marker R may include a radioactive isotope, a fluorescent dye, or another type of labeling material. As the marker R is coupled to the first binding material 105a, the optical unit 230 radiates light toward the tip 110, and the presence of the detection target material T included in the sample S and the amount of the detection target material T included in the sample S may be determined using the intensity of light generated by the marker R. A detailed description thereof will be provided later.

Washing solutions 106a through 109a are stored in the washing wells 106 to 109, respectively. The washing well may be one, but may be provided with two or more, and is not particularly limited thereto. FIG. 1 shows a cartridge 100 with four washing wells 106 to 109.

The washing solutions 106a through 109a may include distilled water and buffers other than distilled water depending on the characteristics of the detection target material T to be detected, and different types of distilled water and buffers other than distilled water may be independently stored in different washing wells 106 to 109.

In one embodiment of the present disclosure, the buffers other than distilled water may include a PBS-based solution, more specifically, a mixed solution of Na2HPO4, KH2PO4, NaCl, C58H11-O26 solution (Tween-20), and 5-chloro-2-methyl-1,2-thiazol-3-one solution (Proclin 300). The cartridge 100 according to an embodiment of the present disclosure includes buffers other than distilled water, so that unreacted biological material remaining on the lower surface of the tip 110 may be more effectively removed, thereby improving the accuracy of analysis.

A tag capable of specifying a specimen from which the sample S has been collected may be printed on the outer surface of the cartridge 100, and analysis may be performed by specifying the specimen from which the sample S has been collected through the process of recognizing the tag. Thus, the analysis result of the cartridge 100 may be processed, stored, and output by matching the specimen specified by tag recognition.

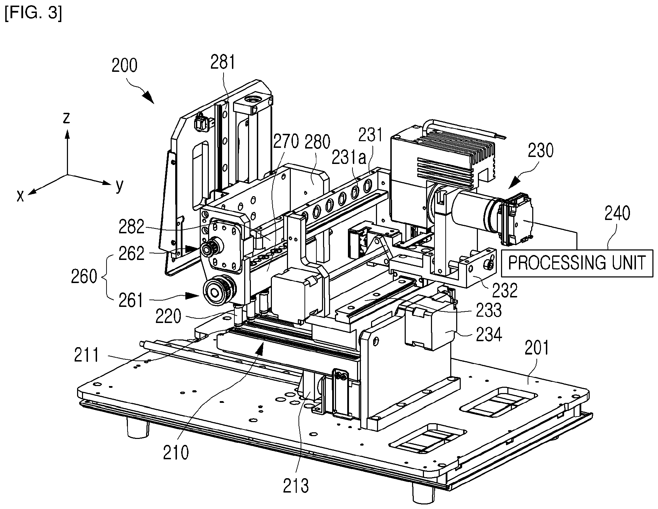

Next, the multiple biomarker simultaneous analysis apparatus 200 according to an embodiment of the present disclosure will be described in detail with reference to FIGS. 3 through 7.

The multiple biomarker simultaneous analysis apparatus 200 is equipped with the cartridge 100 described above, performs analysis of the sample S injected into the cartridge 100, and determines whether the detection target material T is present in the sample S.

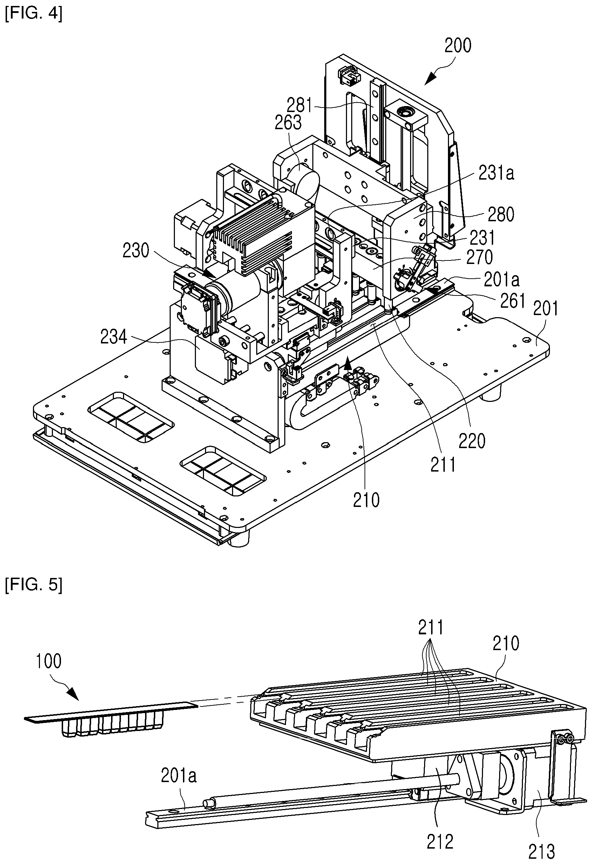

Referring to FIGS. 3 and 4, the multiple biomarker simultaneous analysis apparatus 200 includes a cartridge mounting portion 210, a tip coupling portion 220, an optical unit 230, a processing unit 240, a cartridge mounting portion moving portion, and a tip coupling portion moving portion 260, a tip coupling portion mounting base 270, and a mounting base support portion 280.

The cartridge mounting portion 210 is formed with a seating portion 211 on which the cartridge 100 is placed. The number of seating portions 211 formed in the cartridge mounting portion 210 may be one, but it is also possible to have two or more, and FIG. 2 shows an embodiment in which six seating portions 211 are formed.

One cartridge 100 may be installed in one seating portion 211, and in the case of the cartridge mounting portion 210 in which a plurality of seating portions 211 are formed, a plurality of cartridges 100 may be analyzed at a time. A detailed description thereof will be provided later.

The cartridge mounting portion 210 is movable by the cartridge mounting portion moving portion.

Specifically, the cartridge mounting portion 210 is installed while being spaced apart from a bottom surface 201 of the multiple biomarker simultaneous analysis apparatus 200 by a predetermined distance, and the cartridge mounting portion support 212 is formed between the bottom surface 201 and the cartridge mounting portion 210 so that the cartridge mounting portion 210 may be supported.

A first moving rail 201a for movement of the cartridge mounting portion 210 is formed on the bottom surface 201, and the cartridge mounting portion support 212 is coupled to the first moving rail 201a so as to move along the first moving rail 201a.

In addition, the cartridge mounting portion support 212 is connected to a rotation shaft of a first driving motor 213 that provides a driving force for the movement of the cartridge mounting portion 210, and when the rotation of the first driving motor 213 is made, the cartridge mounting portion support 212 is movable along the first moving rail 201a.

The moving direction of the cartridge mounting portion 210 according to the rotation of the first driving motor 213 may be a direction parallel to the ground. However, embodiments of the present disclosure are not limited thereto, and it is also possible for the cartridge mounting portion 210 to move in the left and right directions while forming a predetermined angle with respect to the ground.

The tip coupling portion 220 is coupled to the tip 110 and the punching tip 120 described above. More specifically, the tip coupling portion 220 is inserted into the tip coupling hole 110a formed at the rear end of the tip 110, thereby being coupled to the tip 110. In addition, the tip coupling portion 220 is inserted into the punching tip coupling hole 120a formed at the rear end of the punching tip 120, thereby being coupled to the punching tip 120.

The tip coupling portion 220 is installed in such a way that a part of the front end of the tip coupling portion 220 is exposed to the outside and the other part that is not exposed is inserted into the tip coupling portion mounting base 270.

The mounting base support portion 280 is coupled to the outside of the tip coupling portion mounting base 270.

In the multiple biomarker simultaneous analysis apparatus 200, a second moving rail 281 for movement of the mounting base support portion 280 is extended in a vertical direction, and the mounting base support portion 280 is coupled to the second moving rail 281 so as to move along the second moving rail 281.

In addition, the mounting base support portion 280 is connected to a rotation shaft of a second driving motor 282 that provides a driving force for movement of the mounting base support portion 280, and when the second driving motor 282 rotates, the mounting base support portion 280 is movable along the second moving rail 281.

When the mounting base support portion 280 moves, the tip coupling portion mounting base 270 coupled to the mounting base support portion 280, and the tip coupling portion 220 installed on the tip coupling portion mounting base 270 also move together, and the movement direction of the mounting base support portion 280 according to the rotation of the second driving motor 282 may be a direction perpendicular to the ground. However, embodiments of the present disclosure are not limited thereto, and it is also possible to move the mounting base support portion 280 in the vertical direction while forming a predetermined angle with respect to the ground.

That is, the moving direction of the tip coupling portion 220 may be perpendicular to the moving direction of the cartridge 100.

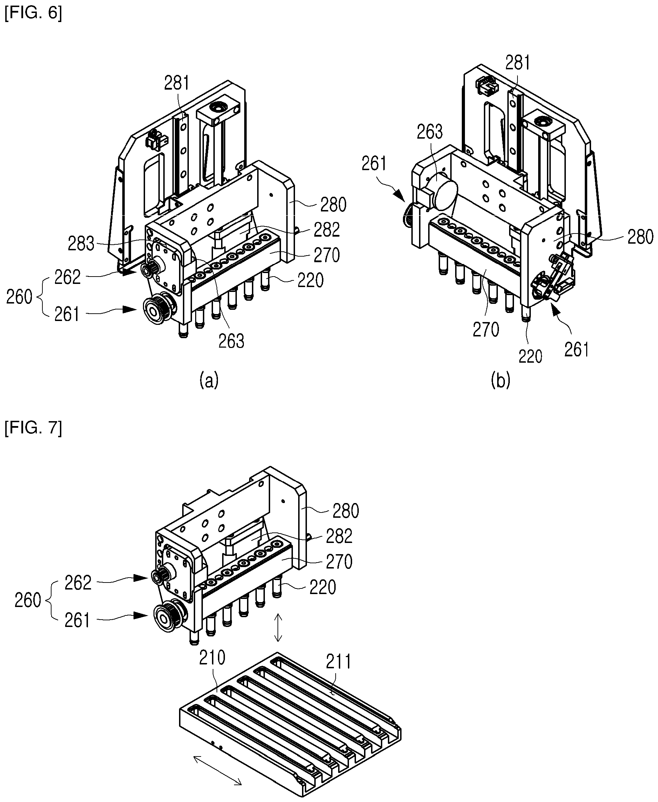

The tip coupling portion moving portion 260 for moving the tip coupling portion 220 may be formed in the mounting base support portion 280. Referring to FIG. 6, the tip coupling portion moving portion 260 may be formed on the side of the mounting base support portion 280 and may include a first tip coupling portion moving portion 261 and a second tip coupling portion moving portion 262.

The first tip coupling portion moving portion 261 is a portion for rotational movement of the tip coupling portion 220.

The first tip coupling portion moving portion 261 is coupled to the tip coupling portion mounting base 270 while passing through the mounting base support portion 280 from both sides of the mounting base support portion 280.

The first tip coupling portion moving port 261 formed on both sides of the mounting base support portion 280 each include a third driving motor (not shown), and as the third driving motor (not shown) rotates, the tip coupling portion mounting base 270 coupled thereto may be rotated.

When the tip coupling portion mounting base 270 rotates, the tip coupling portion 220 installed here also rotates together, and more specifically, the tip coupling portion 220 may be rotated from a position perpendicular to the ground to a position parallel to the ground.

When the tip coupling portion 220 is rotated to the position parallel to the ground by rotation of the third driving motor (not shown), the lower surface of the tip 110 coupled to the tip coupling portion 220 is located on an optical path on which light is radiated by the optical unit 230. Thus, because analysis is performed in a state in which the lower surface of the tip 110 is exposed to outside air instead of in a state supported on a sample or reagent, interference by external factors such as contamination and residues is not made. Thus, a more accurate optical signal may be detected.

The second tip coupling portion moving portion 262 is a portion for vibrating the tip coupling portion 220 in the vertical direction.

The second tip coupling portion moving portion 262 is installed on one side of the mounting base support portion 280. To this end, a mounting groove 283 having a length longer than the length of the second tip coupling portion moving portion 262 is formed in the mounting base support portion 280.

The second tip coupling portion moving portion 262 is coupled to a rotation shaft of the fourth driving motor 263 installed to face the mounting base support portion 280 interposed therebetween, and when the rotation of the fourth driving motor 263 is made, the second tip coupling portion moving portion 262 may vibrate in the vertical direction along the mounting groove 283.

In other words, the vertical length of the mounting groove 283 is longer than the vertical length of the second tip coupling portion moving portion 262, and the mounting base support portion 280 can vibrate in the vertical direction by using a difference between the vertical length of the mounting groove 283 and the vertical length of the second tip coupling portion 262 as an amplitude.

When the mounting base support portion 280 vibrates in the vertical direction, the tip coupling portion mounting base 270 coupled to the mounting base support portion 280, and the tip coupling portion 220 installed in the tip coupling portion mounting base 270 vibrate in the vertical direction.

The tip 110 may vibrate in the vertical direction with a predetermined amplitude in the well by vibration in the vertical direction. Thus, reaction between the binding materials coupled to the lower surface of the tip 110 and the detection target material, reaction between the detection target material T and the binding material in the reaction sample 105c, and the washing of the unreacted biological material remaining on the lower surface of the tip 110 may be performed more effectively.

The optical unit 230 includes a radiator for radiating light toward the outside, and a receiving portion for receiving the light entering the inside.

The tip coupling portion 220 is rotated by the first tip coupling portion moving portion 261, and the tip 110 coupled to the tip coupling portion 220 is rotated together.

At this time, the lower surface of the tip 110 is located on the optical path on which light is radiated by the optical unit 230. For example, a binder bound in the order of the first bonding material, the detection target material, and the second bonding material is located on the lower surface of the tip 110. Here, the marker R such as a radioactive isotope, fluorescent material, *etc.,* is bound to the first binding material, and the first binding material generates a specific optical signal according to the type of the marker R by light radiation of the optical unit 230.

The optical unit 230 may receive the specific optical signal, and the processing unit 240 determines the presence of the detection target material T in the sample S and the amount of the detection target material T included in the sample S according to the intensity of the received optical signal.

An intermediate portion 231 through which a through hole 231 is formed, is further formed between the optical unit 230 and the tip coupling portion 220.

The number of through holes 231a may be the same as the number of seating portions 211 formed in the cartridge mounting portion 210, and the intermediate portion 231 having six through holes 231a formed therein is shown in FIG. 3.

The light radiated by the optical unit 230 passes through the through hole 231a of the intermediate portion 231, and the optical signal generated on the lower surface of the tip 110 according to the light radiation also passes through the through hole 231a and is received by the optical unit 230.

Only the optical signal passing through the through hole 231a is received by the optical unit 230, and as analysis is performed using the received optical signal, interference caused by light incident from the outside may be minimized.

The optical unit 230 is installed to be spaced apart from the bottom surface 201 by a predetermined distance, and an optical unit support 232 is installed between the bottom surface 201 and the optical unit 230, and the optical unit support 232 is movable along the third moving rail 233 in a direction parallel to the ground. To this end, the optical unit support 232 is coupled to a fifth driving motor 234, so that the optical unit support 232 may move along the third moving rail 233 according to the rotation of the fifth driving motor 234.

More specifically, the optical unit 230 is movable when the optical unit 230 and the through hole 231a are aligned with each other so that light radiated from the optical unit 230 passes through the through hole 231a formed in the intermediate portion 231. All of the plurality of tips 110 coupled to the plurality of tip coupling portions 220 may be analyzed according to the movement of the optical unit 230.

The moving direction of the multiple biomarker simultaneous analysis apparatus 200 according to an embodiment of the present disclosure is summarized as follows.

As shown in FIG. 3, if a three-axis coordinate system consisting of an x-axis, a y-axis, and a z-axis is described as an example, the cartridge mounting portion 210 is movable along the y-axis, and the vertical movement of the tip coupling portion 270 is movable along the z-axis, and the optical unit 230 is movable along the x-axis. The movement of the cartridge mounting portion 210, the vertical movement of the tip coupling portion mounting base 270, and the movement of the optical unit 230 may be perpendicular to each other. However, embodiments of the present disclosure are not particularly limited thereto.

The processing unit 240 determines whether the detection target material T is present in the sample S injected into the cartridge 100 using the light received by the optical unit 230 and the amount of the detection target material T included in the sample S.

The processing unit 240 may be a microprocessor (MCU) or a computer having data operation and processing functions, but is not particularly limited thereto as long as the processing unit 240 has data operation and processing functions.

FIGS. 23 through 25 will be described as an example.

When the detection target material T is present in the sample S, the detection target material T is specifically bound to the binding material formed on the tip 110, and the detection target material T is also specifically bound to the binding material contained in the reaction sample 105c.

Thus, a binder bound in the order of the first binding material, the detection target material, and the second binding material is formed. A marker R, such as a radioactive isotope, a fluorescent material, *etc.* is bound to the first binding material, and the first binding material generates a specific optical signal by light radiation.

When the detection target material T is included in the sample S, a specific optical signal may be received by the optical unit 230 by light radiation, and the processing unit 240 may determine the presence or absence of the detection target material T in the sample S and the amount of the detection target material T contained in the sample S using the positions of different binding materials coupled to the lower surface of the tip 110 and the intensity of the optical signal at the corresponding positions.

FIG. 23 shows a state in which an optical signal having a predetermined intensity or higher is not detected at positions where other binding materials are formed except for positions where reference point markers are formed. The processing unit 240 may determine that the detection target material T is not present in the corresponding sample.

In addition, FIG. 24 shows a state in which an optical signal having a high intensity is detected at a position where the third binding material 113 that is an AFP antibody and the fourth binding material 114 that is a CEA antibody are formed and an optical signal having a low intensity is detected at a position where the fifth binding material 115 that is a PSA antibody and the sixth binding material 116 that is a CA125 antibody are formed. In addition, FIG. 24 shows a state in which an optical signal having a very low intensity is detected at a position where the second binding material 111 that is a CA19-9 antibody is formed.

The processing unit 240 may determine that the detection target material of CA19-9, AFP, CEA, PSA and CA125 is present in the corresponding sample, but may determine that AFP and CEA are more than PSA and CA125, and that CA19-9 is less than PSA and CA125.

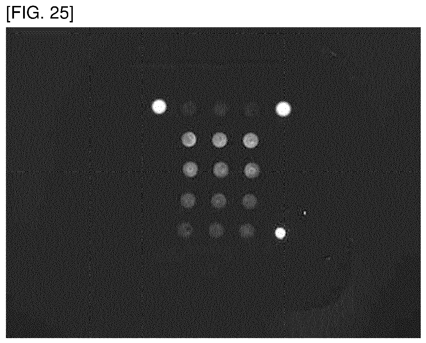

In FIG. 25, an optical signal having a low intensity is detected at the position where the third binding material 113 that is an AFP antibody and the fourth binding material 114 that is a CEA antibody are formed, and an optical signal having a very low intensity is detected at the position where the fifth binding material 115 that is a PSA antibody and the sixth binding material 116 that is a CA125 antibody are formed. In addition, almost no optical signal is detected at the position where the second binding material 111 that is a CA19-9 antibody is formed.

The processing unit 240 may determine that CA19-9 is not present in the corresponding sample, and may determine that AFP, CEA, PSA, and CA125 are present, but the processing unit 240 may determine that AFP and CEA are more than PSA and CA125. Also, the processing unit 240 may determine that the amounts of AFP and CEA in the corresponding sample of FIG. 25 are less than the amounts of AFP and CEA in the corresponding sample of FIG. 24 using the received light intensity.

As such, in the multiple biomarker simultaneous analysis apparatus 200 according to an embodiment of the present disclosure, multiple biomarkers are coupled to one tip 110, and an optical signal at a position where a corresponding biomarker is coupled to the tip 110, is analyzed so that the presence or absence of the biomarker in the sample S may be determined.

In addition, in the multiple biomarker simultaneous analysis apparatus 200 according to an embodiment of the present disclosure, a plurality of cartridges 100 may be placed in one cartridge mounting portion 210, and the presence or absence of a biomarker in the sample S for a plurality of specimens may also be determined in one analysis operation.

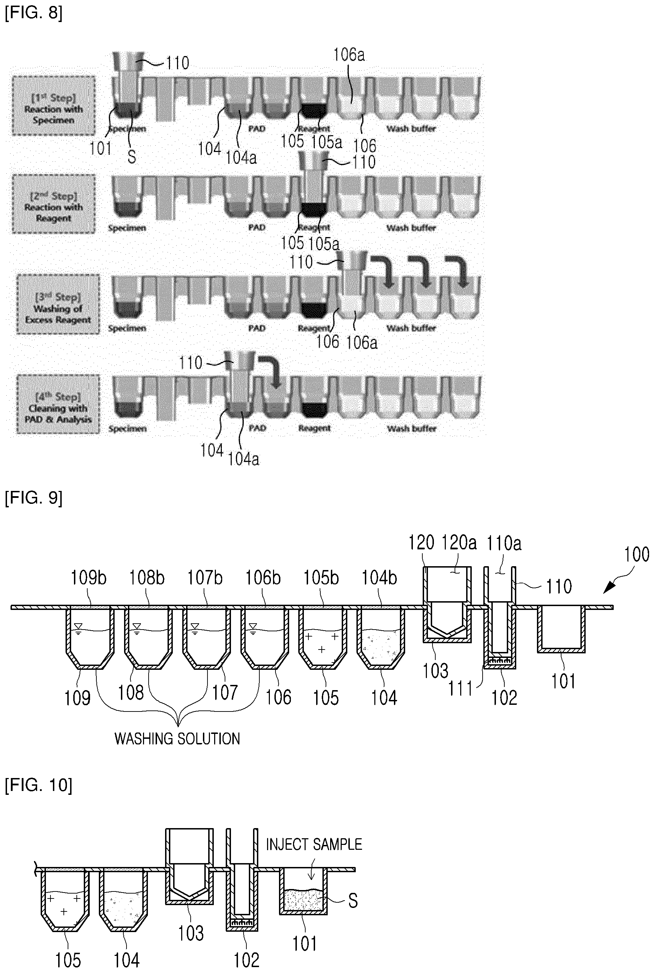

Hereinafter, a multiple biomarker simultaneous analysis method using the multiple biomarker simultaneous analysis apparatus 200 will be described in detail with reference to FIGS. 9 through 21.

First, the cartridge 100 is installed on the seating portion 211 of the cartridge mounting portion 210.

Next, the sample S is injected into the sample well 101 of the cartridge 100 (FIG. 10).

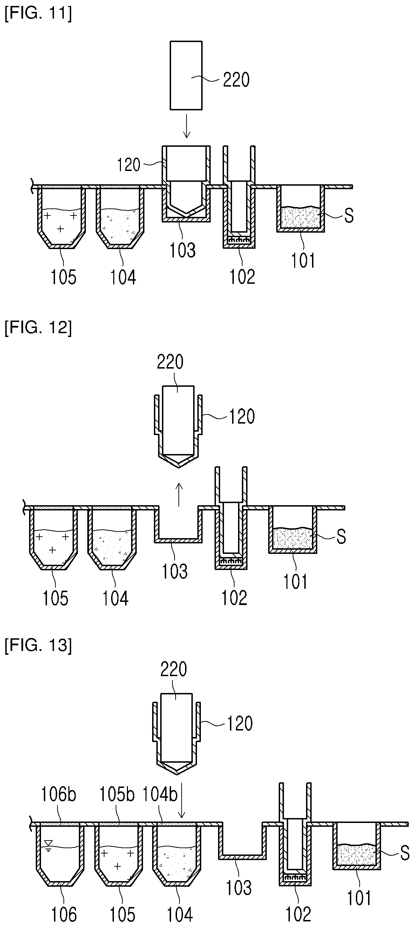

Next, the first driving motor 213 rotates so that the cartridge mounting portion 210 moves along the y-axis direction, and the punching tip well 103 of the cartridge 100 and the tip coupling portion 220 are aligned in a line with each other in the vertical direction (in the z-axis direction) (FIG. 11).

Next, the second driving motor 282 rotates so that the mounting base support portion 280 moves downward along the z-axis direction, and the punching tip 120 provided in the punching tip well 103 and the tip coupling portion 220 are coupled to each other (FIG. 12).

Next, the first driving motor 213 rotates so that the cartridge mounting portion 210 moves along the y-axis direction, and each of the moisture absorption pad well 104, the reaction well 105 and one or more washing wells 106 through 109 of the cartridge 100 is aligned in a line with the tip coupling portion 220 in the vertical direction.

Next, the second driving motor 282 rotates so that the mounting base support portion 280 moves downward along the z-axis direction, and each of sealing portions 104b, 105b, 106b, 107b, 108b, and 109b for sealing the moisture absorption pad well 104, the reaction well 105, and one or more washing wells 106 through 109 is perforated by the punching tip 120 (FIG. 13).

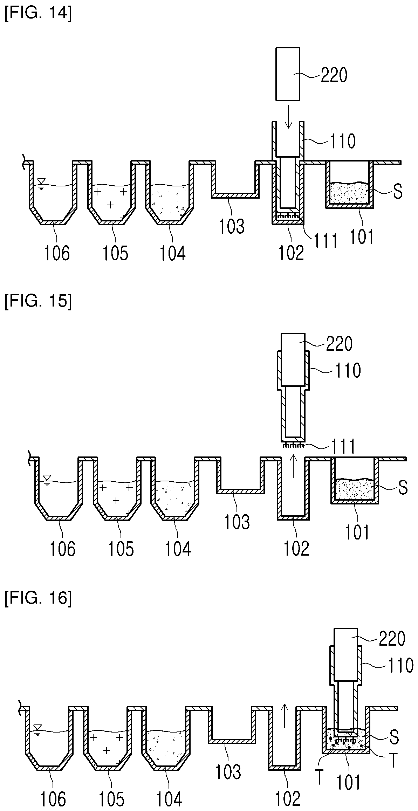

Next, the tip coupling portion 220 and the punching tip 120 are separated, and the first driving motor 213 rotates so that the cartridge mounting portion 210 moves along the y-axis direction to combine the tip well 102 and the tip. The portions 220 are aligned in a vertical direction (FIG. 14).

Next, the second driving motor 282 rotates so that the mounting base support portion 280 moves downward along the z-axis direction, and the tip 110 provided in the tip well 102 and the tip coupling portion 220 are coupled to each other (FIG. 15).

Next, the first driving motor 213 rotates so that the cartridge mounting portion 210 moves along the y-axis direction, and the sample well 101 and the tip coupling portion 220 are aligned in a line in the vertical direction.

Next, the second driving motor 282 rotates so that the mounting base support portion 280 moves downward along the z-axis direction, and the tip 110 coupled to the tip coupling portion 220 is supported on the sample S of the sample well 101 (FIG. 16).

In this process, the detection target material T included in the sample S and the binding material that is specifically bound to the detection target material T and coupled to the lower surface of the tip 110 are bound.

In addition, in this process, the fourth driving motor 263 rotates, and accordingly, the mounting base support portion 280, the tip coupling portion mounting base 270, and the tip coupling portion 220 vibrate in the vertical direction with a predetermined amplitude.

That is, as the lower surface of the tip 110 vibrates in the vertical direction while being supported on the sample S, the binding material coupled to the lower surface of the tip 110 and the detection target material T contained in the sample S may be effectively bound. However, the vertical vibration movement of the tip coupling portion 220 is not essential, and this process may be omitted.

Next, the first driving motor 213 rotates so that the cartridge mounting portion 210 moves along the y-axis direction, and the reaction well 105 and the tip coupling portion 220 are aligned in a line in the vertical direction.

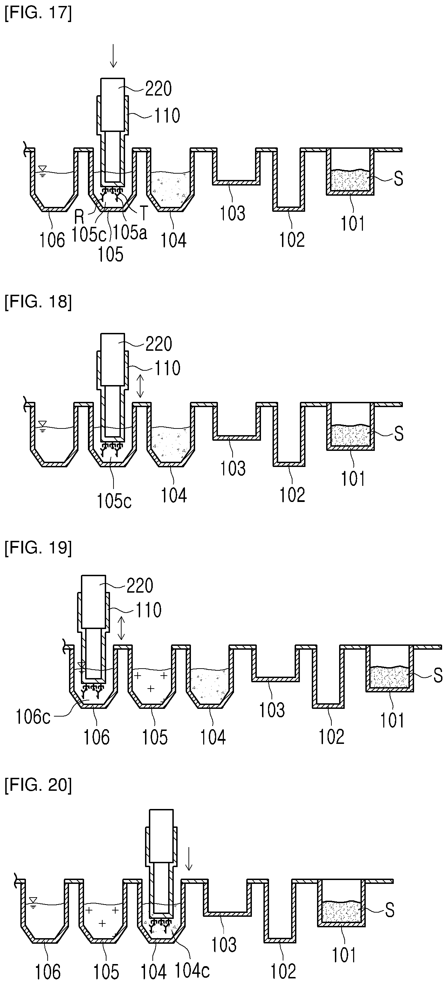

Next, the second driving motor 282 rotates so that the mounting base support portion 280 moves downward along the z-axis direction, and the lower surface of the tip 110 is supported on the reaction sample 105c provided in the reaction well 105 (FIG. 17).

In this process, the first binding material 105a included in the reaction sample 105c is specifically bound to a binding material-detection target material binder bound to the lower surface of the tip 110 (more specifically, is specifically bound to the detection target material).

In this process, the fourth driving motor 263 rotates, and accordingly, the mounting base support 280, the tip coupling portion mounting base 270, and the tip coupling portion 220 vibrate in the vertical direction with a predetermined amplitude (FIG. 18).

That is, as the lower surface of the tip 110 vibrates in the vertical direction while being supported on the reaction sample 105c, the binding material-detection target material binder coupled to the lower surface of the tip 110 and the first binding material 105a included in the reaction sample 105c may be effectively bound.

Next, the first driving motor 213 rotates so that the cartridge mounting portion 210 moves along the y-axis direction, and any one washing well 106 of one or more washing wells 106 through 109 and the tip coupling portion 220 are aligned in a line in the vertical direction.

Next, the second driving motor 282 rotates so that the mounting base support portion 280 moves downward along the z-axis direction, and the lower surface of the tip 110 is supported in the washing solution 106a provided in the washing well 106 (FIG. 19).

In this process, the unreacted biological material remaining on the lower surface of the tip 110 may be washed by the washing solution 106a, and as a result of the washing, for example, only a first binding material-detection target material-second binding material binder may be bound to the lower surface of the tip 110. Thus, the problem of inaccurate analysis due to the occurrence of interference of the optical signal due to the unreacted biological material is prevented.

In this process, the fourth driving motor 263 rotates, and accordingly, the mounting base support portion 280, the tip coupling portion mounting base 270, and the tip coupling portion 220 vibrate in the vertical direction with a predetermined amplitude.

That is, as the lower surface of the tip 110 vibrates in the vertical direction while being supported in the washing solution 106a, the unreacted biological material remaining on the lower surface of the tip 110 may be effectively washed.

Next, the first driving motor 213 rotates so that the cartridge mounting portion 210 moves along the y-axis direction so that the moisture absorption pad well 104 and the tip coupling portion 220 are aligned in a line in the vertical direction.

Next, the second driving motor 282 rotates so that the mounting base support portion 280 moves downward along the z-axis direction, and the lower surface of the tip 110 is inserted into the moisture absorption pad 104a provided in the moisture absorption pad well 104 (FIG. 20).

In this process, moisture and the unreacted biological material remaining on the lower surface of the tip 110 may be separated from the tip 110.

Next, the tip coupling portion mounting base 270 is rotated according to the rotation of the third driving motor (not shown), and accordingly, the lower surface of the tip 110 is located on the optical path on which light is radiated by the optical unit 230.

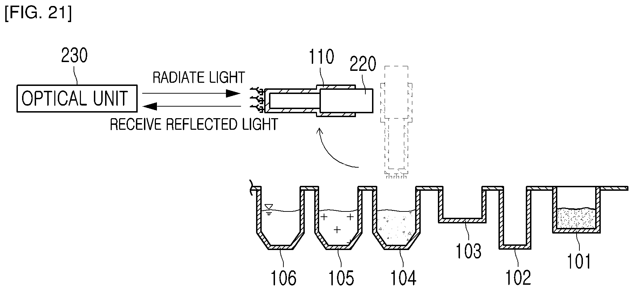

Next, the optical unit 230 radiates light toward the lower surface of the tip 110, and a specific optical signal generated as the radiated light reaches the marker R, is received by the optical unit 230 (FIG. 21).

Next, the processing unit 240 may determine the presence of the detection target material T, for example, corresponding to a portion of the tip 110 having an intensity greater than or equal to a predetermined intensity level using the intensity of the optical signal received by the optical unit 230, and further, may determine the amount of the detection target material T.

When a multiple biomarker simultaneous analysis apparatus of the present disclosure is used, analysis errors caused by components other than a sample can be minimized through a process in which a detection target material is coupled to the lower end of a tip and moves, so that time and cost required for a detection process of the detection target material can be reduced.

Furthermore, as compared to an analysis apparatus according to the related art that detects an optical signal generated within a well of a cartridge, in the analysis apparatus according to the present disclosure, an optical signal generated at the tip outside the well of the cartridge can be detected. Accordingly, in the analysis apparatus according to the present disclosure, because the optical signal is less or not subject to complete interference by external factors such as contamination and residues, a more accurate optical signal can be detected.

## Claims

1. A multiple biomarker simultaneous analysis apparatus comprising:
a cartridge (100) comprising
a plurality of wells and
a tip (110), and
wherein the plurality of wells comprise a sample well (101) configured to accommodate a sample (S) containing a detection target material (T), and a reaction well (105) configured to accommodate a first binding material (105a), which is specifically configured to be bound to the detection target material (T) and to which a marker (R) is coupled, and the tip (110) is configured to be inserted into the plurality of wells and a second binding material (111) that is specifically configured to be bound to the detection target material (T) is coupled to one end of the tip (110);
a cartridge mounting portion (210) in which the cartridge (100) is installed;
a tip coupling portion (220) coupled to the tip (110) and movable on a predetermined movement trajectory while being coupled to the tip (110);
an optical unit (230) configured to radiate light toward the tip (110) and receive light generated from the tip (110) according to light radiation;
a processing unit (240) configured to determine whether the detection target material (T) is present in the sample (S) according to a predetermined method using the light received by the optical unit (230);
a cartridge mounting portion moving portion which is coupled to the cartridge mounting portion (210) and configured to move the cartridge mounting portion (210) so that the plurality of wells of the cartridge (100) and the tip coupling portion (220) are aligned with each other and configured to move the cartridge mounting portion in a direction perpendicular to the vertical direction; and
a tip coupling portion moving portion (260) which is coupled to the tip coupling portion (220) and configured to move the tip coupling portion (220) on the predetermined movement trajectory;
a tip coupling portion mounting base (270) in which the tip coupling portion (220) is installed;
a mounting base support portion (280) which is installed on a moving rail (281) having one end extending in a vertical direction and coupled to an outside of the tip coupling portion mounting base (270),
wherein the tip coupling portion moving portion (260) comprises:
a first tip coupling portion moving portion (261), which is installed on the tip coupling portion mounting base (270) and is configured to provide a driving force so that the tip coupling portion mounting base (270) rotates between a position where the tip coupling portion (220) installed on the tip coupling portion mounting base (270) is aligned in a line with the plurality of wells of the cartridge (100) in the vertical direction and a position where the tip coupling portion (220) is aligned in a line with an optical path on which light is radiated by the optical unit (230); and
a second tip coupling portion moving portion (262), which is installed on the mounting base support portion (280) and is configured to provide a driving force so that the tip coupling portion mounting base (270) vibrates in the vertical direction with a predetermined amplitude,
wherein the position where the tip coupling portion installed on the tip coupling portion mounting base is aligned in a line with the plurality of wells is a position perpendicular to ground, and the position where the tip coupling portion is aligned in a line with an optical path is a position parallel to the ground, and
wherein the cartridge mounting portion comprises a plurality of seating portions in which the cartridge is installed, the plurality of seating portions being arranged in parallel to each other, and the tip coupling portion is formed in a number matching a number of the seating portions one-to-one.

2. The multiple biomarker simultaneous analysis apparatus of claim 1, wherein the cartridge (100) further comprises:
a tip well (102) into which the tip (110) is inserted; and
at least one absorption pad well (104) in which an absorption pad (104a) is installed;
at least one washing well (106, 107, 108, and 109) in which a washing solution is accommodated; and
a punching tip well (103) into which a punching tip (120) for punching sealing portions (104b, 105b, 106b, 107b, 108b, and 109b) of the absorption pad well (104), the reaction well (105), and the at least one washing well (106, 107, 108, and 109).

3. The multiple biomarker simultaneous analysis apparatus of claim 2, wherein the tip coupling portion (220) is selectively coupled to the tip (110) or the punching tip (120).

4. The multiple biomarker simultaneous analysis apparatus of claim 1, wherein at least one binding material that is specifically bound to different detection target materials from the detection target material (T) is further coupled to a lower surface of the tip (110) to which the second binding material (111) is coupled.

5. The multiple biomarker simultaneous analysis apparatus of claim 1, further comprising an intermediate portion (231), which is installed between the optical unit (230) and the tip coupling portion mounting base (270) and has at least one through hole (231a) formed on an optical path on which light is radiated by the optical unit (230).

6. The multiple biomarker simultaneous analysis apparatus of claim 4, wherein the processing unit (240) is configured to determine whether a plurality of detection target materials (T) are present in the sample (S) using a number of combinations of intensities of light received by the optical unit (230) for different detection target materials.

7. The multiple biomarker simultaneous analysis apparatus of claim 5, wherein the processing unit (240) is configured to determine whether the plurality of detection target materials (T) are present in the sample (S) using positions of binding materials coupled to the lower surface of the tip (110) and the number of combinations of intensities of light received at the positions.

8. The multiple biomarker simultaneous analysis apparatus of claim 5, wherein the processing unit (240) is configured to determine amounts of the plurality of detection target materials (T) contained in the sample (S) using positions of binding materials coupled to the lower surface of the tip (110) and the number of combinations of intensities of light received at the positions.

9. A multiple biomarker simultaneous analysis method using the multiple biomarker simultaneous analysis apparatus of claim 2, the multiple biomarker simultaneous analysis method comprising:
(a) injecting the sample (S) into the sample well (101) of the cartridge (100);
(b) aligning the tip well (102) of the cartridge (100) and the tip coupling portion (220) in a line in a vertical direction;
(c) coupling the tip coupling portion (220) to the tip (110);
(d) aligning the sample well (101) of the cartridge (100) and the tip coupling portion (220) in a line in the vertical direction and injecting the tip (110) into the sample (S) accommodated in the sample well (101);
(e) aligning the reaction well (105) of the cartridge (100) and the tip coupling portion (220) in a line in the vertical direction and injecting the tip (110) into a reaction sample (105c) accommodated in the reaction well (105);
(f) aligning one of the at least one washing well (106, 107, 108, and 109) of the cartridge (100) and the tip coupling portion (220) in a line in the vertical direction and injecting the tip (110) into a washing solution accommodated in the one washing well;
(g) aligning the moisture absorption pad well (104) of the cartridge (100) and the tip coupling portion (220) in a line in the vertical direction and injecting the tip (110) into the moisture absorption pad (104a) provided in the moisture absorption pad well (104);
(h) rotating the tip coupling portion mounting base (270) so that the optical path on which light is radiated by the optical unit (230) and the tip (110) are aligned in a line;
(i) radiating light toward the tip (110) using the optical unit (230) and receiving the light generated from the tip (110) by the optical unit (230); and
(j) determining whether the detection target material (T) is present in the sample (S) using intensity of light received by the optical unit (230), wherein the determining is performed by using the processing unit (240).

10. The multiple biomarker simultaneous analysis method of claim 9, further comprising, after (a) and before (b):
(a1) aligning the punching tip well (103) of the cartridge (100) and the tip coupling portion (220) in a line in the vertical direction;
(a2) coupling the tip coupling portion (220) to the punching tip (120); and
(a3) aligning each of the moisture absorption pad well (104), the reaction well (105), and the at least one washing well (106, 107, 108, and 109) of the cartridge (100) with the tip coupling portion (220) in a line in the vertical direction and punching the sealing portion (104b) of the moisture absorption pad well (104), the sealing portion (105b) of the reaction well (105), and the sealing portions (106b, 107b, 108b, and 109b) of the at least one washing well (106, 107, 108, and 109).

11. The multiple biomarker simultaneous analysis method of claim 9, wherein any one or more of (e), (f), and (g) further comprise vibrating the tip coupling portion (220) in the vertical direction with a predetermined amplitude while being injected into a well of the cartridge (100).

## Patentansprüche

1. Einrichtung zur gleichzeitigen Analyse mehrerer Biomarker, umfassend:
eine Kartusche (100), umfassend
eine Vielzahl von Bohrungen und
eine Spitze (110) und
wobei die Vielzahl von Bohrungen eine Probenbohrung (101), die dazu konfiguriert ist, eine Probe (S) aufzunehmen, die ein Erkennungszielmaterial (T) enthält, und eine Reaktionsbohrung (105) umfasst, die konfiguriert ist, um ein erstes Bindematerial (105a) aufzunehmen, das speziell dazu konfiguriert ist, an das Erkennungszielmaterial (T) gebunden zu werden, und an das ein Marker (R) gekoppelt ist, und die Spitze (110) konfiguriert ist, um in die Vielzahl von Bohrungen eingeführt zu werden, und ein zweites Bindematerial (111), das speziell dazu konfiguriert ist, an das Erkennungszielmaterial (T) gebunden zu werden, an ein Ende der Spitze (110) gekoppelt ist;
einen Kartuschenmontageabschnitt (210), in dem die Kartusche (100) installiert ist;
einen Spitzenkopplungsabschnitt (220), der mit der Spitze (110) gekoppelt ist und auf einer vorgegebenen Bewegungsbahn beweglich ist, während er mit der Spitze (110) gekoppelt ist;
eine optische Einheit (230), die konfiguriert ist, um Licht in Richtung der Spitze (110) abzustrahlen und von der Spitze (110) erzeugtes Licht entsprechend der Lichtabstrahlung zu empfangen;
eine Verarbeitungseinheit (240), die dazu konfiguriert ist, unter Verwendung des von der optischen Einheit (230) empfangenen Lichts gemäß einem vorgegebenen Verfahren zu bestimmen, ob das Erkennungszielmaterial (T) in der Probe (S) vorhanden ist;
einen Kartuschenmontageabschnitt-Bewegungsabschnitt, der mit dem Kartuschenmontageabschnitt (210) gekoppelt ist und konfiguriert ist, um den Kartuschenmontageabschnitt (210) so zu bewegen, dass die Vielzahl von Bohrungen der Kartusche (100) und der Spitzenkopplungsabschnitt (220) miteinander ausgerichtet sind, und konfiguriert ist, um den Kartuschenmontageabschnitt in einer Richtung senkrecht zur vertikalen Richtung zu bewegen; und
einen Spitzenkopplungsabschnitt-Bewegungsabschnitt (260), der mit dem Spitzenkopplungsabschnitt (220) gekoppelt ist und konfiguriert ist, um den Spitzenkopplungsabschnitt (220) auf der vorgegebenen Bewegungsbahn zu bewegen;
eine Montagebasis (270) für den Spitzenkopplungsabschnitt, in der der Spitzenkopplungsabschnitt (220) installiert ist;
einen Montagebasis-Halterungsabschnitt (280), der auf einer beweglichen Schiene (281) installiert ist, die ein Ende aufweist, das sich in vertikaler Richtung erstreckt und mit einer Außenseite der Montagebasis (270) des Spitzenkopplungsabschnitts gekoppelt ist,
wobei der bewegliche Abschnitt (260) des Spitzenkopplungsabschnitts umfasst:
einen ersten beweglichen Abschnitt (261) des Spitzenkopplungsabschnitts, der auf der Montagebasis (270) des Spitzenkopplungsabschnitts installiert ist und konfiguriert ist, um eine Antriebskraft bereitzustellen, so dass sich die Montagebasis (270) des Spitzenkopplungsabschnitts zwischen einer Position, in der der auf der Montagebasis (270) des Spitzenkopplungsabschnitts installierte Spitzenkopplungsabschnitt (220) in einer Reihe mit der Vielzahl von Bohrungen der Kartusche (100) in der vertikalen Richtung ausgerichtet ist, und einer Position dreht, in der der Spitzenkopplungsabschnitt (220) in einer Reihe mit einem optischen Pfad ausgerichtet ist, auf den Licht von der optischen Einheit (230) abgestrahlt wird; und
einen zweiten beweglichen Abschnitt (262) des Spitzenkopplungsabschnitts, der an dem Montagebasis-Halterungsabschnitt (280) angebracht ist und konfiguriert ist, um eine Antriebskraft bereitzustellen, so dass die Montagebasis (270) des Spitzenkopplungsabschnitts in vertikaler Richtung mit einer vorgegebenen Amplitude vibriert,
wobei die Position, an der der auf der Montagebasis für den Spitzenkopplungsabschnitt installierte Spitzenkopplungsabschnitt in einer Reihe mit der Vielzahl von Bohrungen ausgerichtet ist, eine Position senkrecht zum Boden ist, und die Position, an der der Spitzenkopplungsabschnitt in einer Reihe mit einem optischen Pfad ausgerichtet ist, eine Position parallel zum Boden ist, und
wobei der Kartuschenmontageabschnitt eine Vielzahl von Auflageabschnitten umfasst, in denen die Kartusche installiert ist, wobei die Vielzahl von Auflageabschnitten parallel zueinander angeordnet ist, und der Spitzenkopplungsabschnitt in einer Anzahl ausgebildet ist, die eins zu eins mit der Anzahl der Auflageabschnitte übereinstimmt.

2. Einrichtung zur gleichzeitigen Analyse mehrerer Biomarker nach Anspruch 1, wobei die Kartusche (100) ferner umfasst:
eine Spitzenbohrung (102), in die die Spitze (110) eingesetzt wird; und
mindestens eine Absorptionskissenbohrung (104), in der ein Absorptionskissen (104a) installiert ist;
mindestens eine Waschbohrung (106, 107, 108 und 109), in der eine Waschlösung untergebracht ist; und
eine Stanzspitzenbohrung (103), in die eine Stanzspitze (120) zum Stanzen von Versiegelungsabschnitten (104b, 105b, 106b, 107b, 108b und 109b) der Absorptionskissenbohrung (104), der Reaktionsbohrung (105) und der mindestens einen Waschbohrung (106, 107, 108 und 109) eingesetzt wird.

3. Einrichtung zur gleichzeitigen Analyse mehrerer Biomarker nach Anspruch 2, wobei der Spitzenkopplungsabschnitt (220) selektiv mit der Spitze (110) oder der Stanzspitze (120) gekoppelt ist.

4. Einrichtung zur gleichzeitigen Analyse mehrerer Biomarker nach Anspruch 1, wobei mindestens ein Bindematerial, das spezifisch an andere Erkennungszielmaterialien als das Erkennungszielmaterial (T) gebunden ist, ferner an eine Unterseite der Spitze (110) gekoppelt ist, an die das zweite Bindematerial (111) gekoppelt ist.

5. Einrichtung zur gleichzeitigen Analyse mehrerer Biomarker nach Anspruch 1, ferner umfassend einen Zwischenabschnitt (231), der zwischen der optischen Einheit (230) und der Montagebasis (270) des Spitzenkopplungsabschnitts installiert ist und mindestens ein Durchgangsloch (231a) aufweist, das auf einem optischen Pfad ausgebildet ist, auf den Licht von der optischen Einheit (230) abgestrahlt wird.

6. Einrichtung zur gleichzeitigen Analyse mehrerer Biomarker nach Anspruch 4, wobei die Verarbeitungseinheit (240) konfiguriert ist, um unter Verwendung einer Anzahl von Kombinationen von Lichtintensitäten, die von der optischen Einheit (230) für verschiedene Erkennungszielmaterialien empfangen werden, zu bestimmen, ob eine Vielzahl von Erkennungszielmaterialien (T) in der Probe (S) vorhanden sind.

7. Einrichtung zur gleichzeitigen Analyse mehrerer Biomarker nach Anspruch 5, wobei die Verarbeitungseinheit (240) konfiguriert ist, um anhand der Positionen von Bindematerialien, die mit der Unterseite der Spitze (110) gekoppelt sind, und der Anzahl der Kombinationen von Lichtintensitäten, die an den Positionen empfangen werden, zu bestimmen, ob die Vielzahl von Erkennungszielmaterialien (T) in der Probe (S) vorhanden ist.

8. Einrichtung zur gleichzeitigen Analyse mehrerer Biomarker nach Anspruch 5, wobei die Verarbeitungseinheit (240) konfiguriert ist, um Mengen der Vielzahl von in der Probe (S) enthaltenen Erkennungszielmaterialien (T) unter Verwendung von Positionen von Bindematerialien, die mit der Unterseite der Spitze (110) gekoppelt sind, und der Anzahl von Kombinationen von Lichtintensitäten, die an den Positionen empfangen werden, zu bestimmen.

9. Verfahren zur gleichzeitigen Analyse mehrerer Biomarker unter Verwendung der Einrichtung zur gleichzeitigen Analyse mehrerer Biomarker nach Anspruch 2, wobei das Verfahren zur gleichzeitigen Analyse mehrerer Biomarker umfasst:
(a) Injizieren der Probe (S) in die Probenbohrung (101) der Kartusche (100);
(b) Ausrichten der Spitzenbohrung (102) der Kartusche (100) und des Spitzenkopplungsabschnitts (220) in einer Reihe in vertikaler Richtung;
(c) Koppeln des Spitzenkopplungsabschnitts (220) mit der Spitze (110);
(d) Ausrichten der Probenbohrung (101) der Kartusche (100) und des Spitzenkopplungsabschnitts (220) in einer Reihe in der vertikalen Richtung und Einführen der Spitze (110) in die in der Probenbohrung (101) untergebrachte Probe (S);
(e) Ausrichten der Reaktionsbohrung (105) der Kartusche (100) und des Spitzenkopplungsabschnitts (220) in einer Reihe in der vertikalen Richtung und Einführen der Spitze (110) in eine in der Reaktionsbohrung (105) untergebrachte Reaktionsprobe (105c);
(f) Ausrichten einer der mindestens einen Waschbohrung (106, 107, 108 und 109) der Kartusche (100) und des Spitzenkopplungsabschnitts (220) in einer Reihe in der vertikalen Richtung und Einführen der Spitze (110) in eine Waschlösung, die in der einen Waschbohrung untergebracht ist;
(g) Ausrichten der Bohrung (104) des Feuchtigkeitsabsorptionskissens der Kartusche (100) und des Spitzenkopplungsabschnitts (220) in einer Reihe in der vertikalen Richtung und Einführen der Spitze (110) in das Feuchtigkeitsabsorptionskissen (104a), das in der Bohrung (104) des Feuchtigkeitsabsorptionskissens vorgesehen ist;
(h) Drehen der Montagebasis (270) des Spitzenkopplungsabschnitts, so dass der optische Pfad, auf dem das Licht von der optischen Einheit (230) abgestrahlt wird, und die Spitze (110) in einer Reihe ausgerichtet sind;
(i) Abstrahlen von Licht in Richtung der Spitze (110) unter Verwendung der optischen Einheit (230) und Empfangen des von der Spitze (110) erzeugten Lichts durch die optische Einheit (230); und
(j) Bestimmen, ob das Erkennungszielmaterial (T) in der Probe (S) vorhanden ist, unter Verwendung der von der optischen Einheit (230) empfangenen Lichtintensität, wobei das Bestimmen unter Verwendung der Verarbeitungseinheit (240) durchgeführt wird.

10. Verfahren zur gleichzeitigen Analyse mehrerer Biomarker nach Anspruch 9, ferner umfassend, nach (a) und vor (b):
(a1) Ausrichten der Stanzspitzenbohrung (103) der Kartusche (100) und des Spitzenkopplungsabschnitts (220) in einer Reihe in vertikaler Richtung;
(a2) Koppeln des Spitzenkopplungsabschnitts (220) mit der Stanzspitze (120); und
(a3) Ausrichten jeder der Bohrungen (104) des Feuchtigkeitsabsorptionskissens, der Reaktionsbohrung (105) und der mindestens einen Waschbohrung (106, 107, 108 und 109) der Kartusche (100) mit dem Spitzenkopplungsabschnitt (220) in einer Reihe in der vertikalen Richtung und Stanzen des Versiegelungsabschnitts (104b) der Feuchtigkeitsabsorptionskissenbohrung (104), des Versiegelungsabschnitts (105b) der Reaktionsbohrung (105) und der Versiegelungsabschnitte (106b, 107b, 108b und 109b) der mindestens einen Waschbohrung (106, 107, 108 und 109).

11. Verfahren zur gleichzeitigen Analyse mehrerer Biomarker nach Anspruch 9, wobei eines oder mehrere von (e), (f) und (g) ferner das Vibrieren des Spitzenkopplungsabschnitts (220) in vertikaler Richtung mit einer vorgegebenen Amplitude während des Einführens in eine Bohrung der Kartusche (100) umfassen.

## Revendications

1. Appareil d'analyse simultanée de biomarqueurs multiples comprenant :
une cartouche (100) comprenant
une pluralité de puits et
une pointe (110), et
dans lequel la pluralité de puits comprend un puits à échantillon (101) conçu pour recevoir un échantillon (S) contenant un matériau cible de détection (T), et un puits de réaction (105) conçu pour recevoir un premier matériau de liaison (105a), qui est particulièrement conçu pour être lié au matériau cible de détection (T) et auquel un marqueur (R) est accouplé, et la pointe (110) est conçue pour être insérée dans la pluralité de puits et un second matériau de liaison (111) qui est particulièrement conçu pour être lié au matériau cible de détection (T) est accouplé à une extrémité de la pointe (110) ;
une partie de montage de cartouche (210) dans laquelle la cartouche (100) est installée ;
une partie d'accouplement de pointe (220) accouplée à la pointe (110) et mobile sur une trajectoire de mouvement prédéterminée tout en étant accouplée à la pointe (110) ;
une unité optique (230) configurée pour émettre de la lumière vers la pointe (110) et recevoir de la lumière générée par la pointe (110) selon l'émission de lumière ;
une unité de traitement (240) configurée pour déterminer si le matériau cible de détection (T) est présent dans l'échantillon (S) selon un procédé prédéterminé à l'aide de la lumière reçue par l'unité optique (230) ;
une partie mobile de partie de montage de cartouche qui est accouplée à la partie de montage de cartouche (210) et conçue pour déplacer la partie de montage de cartouche (210) de sorte que la pluralité de puits de la cartouche (100) et la partie d'accouplement de pointe (220) sont alignées l'une sur l'autre et conçues pour déplacer la partie de montage de cartouche dans une direction perpendiculaire à la direction verticale ; et
une partie mobile de partie d'accouplement de pointe (260) qui est accouplée à la partie d'accouplement de pointe (220) et conçue pour déplacer la partie d'accouplement de pointe (220) sur la trajectoire de mouvement prédéterminée ;
une base de montage de partie d'accouplement de pointe (270) dans laquelle la partie d'accouplement de pointe (220) est installée ;
une partie de support de base de montage (280) installée sur un rail mobile (281) dont une extrémité s'étend dans une direction verticale et est accouplée à un extérieur de la base de montage de partie d'accouplement de pointe (270),
dans lequel la partie mobile de partie d'accouplement de pointe (260) comprend :
une première partie mobile de partie d'accouplement de pointe (261), qui est installée sur la base de montage de partie d'accouplement de pointe (270) et est conçue pour fournir une force d'entraînement de sorte que la base de montage de partie d'accouplement de pointe (270) tourne entre une position où la partie d'accouplement de pointe (220) installée sur la base de montage de partie d'accouplement de pointe (270) est alignée sur la pluralité de puits de la cartouche (100) dans la direction verticale et une position où la partie d'accouplement de pointe (220) est alignée sur un chemin optique sur lequel de la lumière est émise par l'unité optique (230) ; et
une seconde partie mobile de partie d'accouplement de pointe (262), installée sur la partie de support de base de montage (280) et conçue pour fournir une force d'entraînement de sorte que la base de montage de partie d'accouplement de pointe (270) vibre dans la direction verticale avec une amplitude prédéterminée,
dans lequel la position où la partie d'accouplement de pointe installée sur la base de montage de partie d'accouplement de pointe est alignée sur la pluralité de puits est une position perpendiculaire au sol, et la position où la partie d'accouplement de pointe est alignée sur un chemin optique est une position parallèle au sol, et
dans lequel la partie de montage de cartouche comprend une pluralité de parties de logement dans lesquelles la cartouche est installée, la pluralité de parties de logement étant disposées parallèlement les unes aux autres, et la partie d'accouplement de pointe est formée dans un nombre correspondant à un nombre de parties de logement un à un.

2. Appareil d'analyse simultanée de biomarqueurs multiples selon la revendication 1, dans lequel la cartouche (100) comprend en outre :
un puits de pointe (102) dans lequel la pointe (110) est insérée ; et
au moins un puits de tampon d'absorption (104) dans lequel un tampon d'absorption (104a) est installé ;
au moins un puits de lavage (106, 107, 108 et 109) dans lequel est logée une solution de lavage ; et
un puits de pointe de perforation (103) dans lequel se trouve une pointe de perforation (120) pour perforer des parties d'étanchéité (104b, 105b, 106b, 107b, 108b et 109b) du puits de tampon d'absorption (104), du puits de réaction (105) et d'au moins un puits de lavage (106, 107, 108 et 109).

3. Appareil d'analyse simultanée de biomarqueurs multiples selon la revendication 2, dans lequel la partie d'accouplement de pointe (220) est sélectivement accouplée à la pointe (110) ou à la pointe de perforation (120).

4. Appareil d'analyse simultanée de biomarqueurs multiples selon la revendication 1, dans lequel au moins un matériau de liaison particulièrement lié à des matériaux cibles de détection différents du matériau cible de détection (T) est en outre accouplé à une surface inférieure de la pointe (110) à laquelle le second matériau de liaison (111) est accouplé.

5. Appareil d'analyse simultanée de biomarqueurs multiples selon la revendication 1, comprenant en outre une partie intermédiaire (231), qui est installée entre l'unité optique (230) et la base de montage de partie d'accouplement de pointe (270) et a au moins un trou traversant (231a) formé sur un chemin optique sur lequel de la lumière est émise par l'unité optique (230).

6. Appareil d'analyse simultanée de biomarqueurs multiples selon la revendication 4, dans lequel l'unité de traitement (240) est configurée pour déterminer si une pluralité de matériaux cibles de détection (T) sont présents dans l'échantillon (S) à l'aide d'un certain nombre de combinaisons d'intensités de lumière reçues par l'unité optique (230) pour différents matériaux cibles de détection.

7. Appareil d'analyse simultanée de biomarqueurs multiples selon la revendication 5, dans lequel l'unité de traitement (240) est configurée pour déterminer si la pluralité de matériaux cibles de détection (T) sont présents dans l'échantillon (S) à l'aide de positions de matériaux de liaison accouplés à la surface inférieure de la pointe (110) et du nombre de combinaisons d'intensités de lumière reçues au niveau des positions.

8. Appareil d'analyse simultanée de biomarqueurs multiples selon la revendication 5, dans lequel l'unité de traitement (240) est configurée pour déterminer des quantités de la pluralité de matériaux cibles de détection (T) contenus dans l'échantillon (S) à l'aide de positions de matériaux de liaison accouplés à la surface inférieure de la pointe (110) et du nombre de combinaisons d'intensités de lumière reçues au niveau des positions.

9. Procédé d'analyse simultanée de biomarqueurs multiples à l'aide de l'appareil d'analyse simultanée de biomarqueurs multiples selon la revendication 2, le procédé d'analyse simultanée de biomarqueurs multiples comprenant :
(a) l'injection de l'échantillon (S) dans le puits à échantillon (101) de la cartouche (100) ;
(b) l'alignement du puits de pointe (102) de la cartouche (100) et de la partie d'accouplement de pointe (220) dans une direction verticale ;
(c) l'accouplement de la partie d'accouplement de pointe (220) à la pointe (110) ;
(d) l'alignement du puits à échantillon (101) de la cartouche (100) et de la partie d'accouplement de pointe (220) dans la direction verticale et l'injection de la pointe (110) dans l'échantillon (S) logé dans le puits à échantillon (101) ;
(e) l'alignement du puits de réaction (105) de la cartouche (100) et de la partie d'accouplement de pointe (220) dans la direction verticale et l'injection de la pointe (110) dans un échantillon de réaction (105c) logé dans le puits de réaction (105) ;
(f) l'alignement d'un de l'au moins un puits de lavage (106, 107, 108 et 109) de la cartouche (100) et de la partie d'accouplement de pointe (220) dans la direction verticale et l'injection de la pointe (110) dans une solution de lavage logée dans le puits de lavage ;
(g) l'alignement du puits de tampon d'absorption d'humidité (104) de la cartouche (100) et de la partie d'accouplement de pointe (220) dans la direction verticale et l'injection de la pointe (110) dans le tampon d'absorption d'humidité (104a) fourni dans le puits de tampon d'absorption d'humidité (104) ;
(h) la rotation de la base de montage de partie d'accouplement de pointe (270) de sorte que le chemin optique sur lequel la lumière est émise par l'unité optique (230) et la pointe (110) sont alignés ;
(i) l'émission de la lumière vers la pointe (110) à l'aide de l'unité optique (230) et la réception de la lumière générée en provenance de la pointe (110) à l'aide de l'unité optique (230) ; et
(j) le fait de déterminer si le matériau cible de détection (T) est présent dans l'échantillon (S) à l'aide d'une intensité de lumière reçue par l'unité optique (230), dans lequel la détermination est réalisée à l'aide de l'unité de traitement (240).

10. Procédé d'analyse simultanée de biomarqueurs multiples selon la revendication 9, comprenant en outre, après (a) et avant (b) :
(a1) l'alignement du puits de pointe de perforation (103) de la cartouche (100) et de la partie d'accouplement de pointe (220) dans la direction verticale ;
(a2) l'accouplement de la partie d'accouplement de pointe (220) à la pointe de perforation (120) ; et
(a3) l'alignement de chacun du puits de tampon d'absorption d'humidité (104), du puits de réaction (105) et de l'au moins un puits de lavage (106, 107, 108 et 109) de la cartouche (100) sur la partie d'accouplement de pointe (220) dans la direction verticale et la perforation de la partie d'étanchéité (104b) du puits de tampon d'absorption d'humidité (104), de la partie d'étanchéité (105b) du puits de réaction (105) et des parties d'étanchéité (106b, 107b, 108b et 109b) de l'au moins un puits de lavage (106, 107, 108 et 109).

11. Procédé d'analyse simultanée de biomarqueurs multiples selon la revendication 9, dans lequel l'un ou plusieurs quelconques des points (e), (f) et (g) comprennent en outre la vibration de la partie d'accouplement de pointe (220) dans la direction verticale avec une amplitude prédéterminée lorsqu'elle est injectée dans un puits de la cartouche (100).
